# EUROPEAN PATENT SPECIFICATION

(11) **EP 1 712 240 B1**
(45) Date of publication and mention of the grant of the patent: **09.09.2015**
(21) Application number: 04807616.0
(22) Date of filing: 22.12.2004
(51) Int. Cl.: A61K 39/395, A61K 47/18, A61K 47/10, A61K 47/34, A61K 9/08, A61K 47/12

(54) **STABLE WATER-BASED MEDICINAL PREPARATION CONTAINING ANTIBODY**
STABILE MEDIZINISCHE ZUBEREITUNG AUF WASSERBASIS MIT ANTIKÖRPER
PREPARATION MEDICAMENTEUSE AQUEUSE STABLE CONTENANT UN ANTICORPS

(30) Priority: 25.12.2003 JP 2003431400
(43) Date of publication of application: 18.10.2006
(73) Proprietor: Kyowa Hakko Kirin Co., Ltd., Tokyo (JP)
(72) Inventor: Ishikawa, Tomoyoshi, Kirin Beer Kabushiki Kaisha, Takasaki-shi, Gunma 3700013 (JP); Ueno, Akihiro, Kirin Beer Kabushiki Kaisha, Takasaki-shi, Gunma 3700013 (JP); Kimura, Satoru, Kirin Beer Kabushiki Kaisha, Takasaki-shi, Gunma 3700013 (JP); Ueki, Yosuke, Greenhouse-Higashinakajima 202, Gifu-shi, Gifu 5008235 (JP)
(74) Representative: Hoffmann Eitle
(86) International application number: PCT/JP2004/019259
(87) International publication number: WO 2005/063291

(56) References cited:
- WO-A1-00/66160
- WO-A1-01/47554
- WO-A1-98/56418
- WO-A1-99/37329
- WO-A1-03/018056
- WO-A1-03/068259
- WO-A2-02/096457
- JP-A- 5 065 233
- JP-A- 5 178 719
- JP-A- 7 502 497
- JP-A- 63 192 724
- JP-A- 2002 504 907
- US-A1- 2001 053 360
- US-A1- 2002 106 371
- US-A1- 2003 180 253
- US-A1- 2004 033 228

## Description

### Technical Field

The present invention relates to a stable liquid medical formulation containing an antibody.

### Background Art

With recent advancement in bioengineering, the full use of DNA recombination technology has enabled mass production of highly pure proteins for medical use. However, proteins differ from conventional chemically-synthesized molecules in terms of their large molecular weights and complicated three-dimensional structures. Stable storage of a protein in a state such that its biological activity is maintained requires special technology established in view of protein physical properties. In the case of a liquid formulation containing a protein which is kept stable, it is required to protect many different functional groups contained in the protein and to maintain its higher-order structure, which is involved in its activity. Antibodies are among the proteins that are medically useful. An example of a method for stabilizing an antibody is a method whereby protein is stabilized by lyophilization as disclosed in JP Patent Publication (Kohyo) No. 2001/503781 A (WO98/22136). However, administration and preliminary work for lyophilized formulations are complicated at clinical sites. Thus, such complicated operation imposes mental and temporal burdens on healthcare professionals. Furthermore, there is concern over the risk of bacterial contamination due to the necessary procedures. In contrast to lyophilized formulations, solution formulations require simple preliminary work before administration. Hence, the risk of bacterial contamination is lowered. At medical sites, protein formulations that are stable in a solution state are desired.

The following documents relate to protein formulations that are stable in a solution state.

Cleland et al., The Development of Stable Protein Preparation: A Close Look at Protein Aggregation, Deamination and Oxidation (Therapeutic Drug Carrier System vol. 10, No. 4, 1993, pp. 307-377) provides a general overview concerning protein degradation and views on measures for reducing protein degradation. However, this document does not disclose that a liquid medical formulation of an antibody contains no salt, nor does it disclose a glutamate buffer or a citrate buffer with a pH between 5.0 and 6.0 used therein.

WO98/56418 discloses a non-lyophilized liquid medical formulation that contains a therapeutically effective antibody, an acetate buffer, a surfactant, and a polyol. However, the document does not disclose that glutamic acid or citric acid is suitable as a buffer agent for maintaining pH.

JP Patent No. 2547556 discloses a γ-globulin formulation containing an acetate buffer and sorbitol. However, the document does not disclose that a buffer agent for maintaining pH contains glutamic acid or citric acid. Furthermore, the document does not disclose that the addition of a surfactant is suitable.

WO97/04801 discloses a lyophilized formulation suitable for subcutaneous administration after it is reconstituted. However, the document discloses neither that the formulation is a non-lyophilized formulation nor that glutamic acid or citric acid with a pH between 5.0 and 6.0 is used as a buffer agent for maintaining pH.

"Stable formulation" contains no ingredients that are toxic to a patient to which the formulation is administered. Furthermore, such formulation contains an active ingredient that retains its chemical and/or physical and/or biological stability during storage through the addition of an additive other than the active ingredient, which does not disturb patient homeostasis as far as is possible. "Additives other than the active ingredient, which do not disturb patient homeostasis as far as is possible" means a substance with safety that has been sufficiently confirmed based on actual past therapeutic performance or a substance with safety that can be sufficiently predicted through evaluation of toxicity to cells or animals or by other methods even when the substance lacks actual past administration performance. Moreover, "to maintain patient homeostasis" means that no additive other than the active ingredient has any biological activity that is unacceptable for a patient and/or that such additive is isotonic (essentially having the same osmotic pressure as that of human blood), if possible.

Protein stability is measured by various analysis methods as generally described in New Protein Purification, Principles and Practice, written by RK Scopes, Springer-Verlag Tokyo, for example. "Chemical stability" can be tested by detecting and quantifying the chemically altered state of a protein. Examples of chemical alterations include: size modification such as clipping that can be evaluated by size exclusion chromatography or SDS-PAGE; changes in electric charge (caused by deamidation, for example) that can be evaluated by ion exchange chromatography; and changes in hydrophilic/hydrophobic states (caused by oxidation, for example) that can be evaluated by hydrophobic chromatography. "Physical stability" means that there is no generation of insoluble foreign matter and/or turbidity and/or aggregates that can be evaluated by visual inspection of color and/or transparency and/or size exclusion chromatography. "Biological stability" can be evaluated by testing binding activity to an antigen. Such binding activity can be evaluated by size exclusion chromatography or ELISA, for example.

Antibodies are included among the proteins that are therapeutically useful. It has been attempted to use an antibody having action of inducing cell death or cytotoxicity through binding to a protein expressed on cell surfaces for treatment of cancer or the like. Currently, monoclonal antibodies such as a chimeric antibody (Rituximab) targeting CD20 that is a receptor existing on cell membranes and a humanized antibody targeting Her2/neu are used for diseases such as malignant tumors, and their therapeutic effects are recognized. As disclosed in WO2003/033538, it is thought that a monoclonal antibody (anti-HLA-DR antibody) against HLA-DR that is a type of major histocompatibility complex (MHC) class II (MHC) molecules is also useful. An antibody is characterized by long blood half-life and high specificity to an antigen, so that it is particularly useful as an antineoplastic agent. For example, in the case of an antibody targeting a tumor-specific antigen, it is inferred that such antibody is accumulated in tumors after administration. Hence, the immune system can be expected to attack cancer cells via complement-dependent cytotoxicity (CDC) or antibody-dependent cellular cytotoxicity (ADCC). Furthermore, previous binding of a medicine such as a radionuclide or a cytotoxic substance to an antibody enables efficient delivery of the bound medicine to a tumor site. Simultaneously, alleviated side effects can also be expected because of a reduction in the amount of medicine that reaches other non-specific tissues. When a tumor-specific antigen has activity of inducing cell death or the like, an antibody having agonistic activity is administered. Furthermore, when a tumor-specific antigen is involved in cell proliferation and survival, an antibody having neutralization activity is administered. Hence, tumor-specific accumulation of an antibody and the arresting of tumor proliferation or degeneration of tumor due to an antibody's activity are expected. As described above, it is thought that an antibody would be appropriate for use as an antineoplastic agent because of its features.

Moreover, a major histocompatibility complex (MHC) class II (MHC) molecule binds to an antigen peptide fragment and then presents such antigen peptide fragment to a helper (CD4+) T cell ("Th" cell) (see Babbin B. et al., Nature (1985), 317, 359-361). It has been reported that a monoclonal antibody specific to the class II MHC molecule is a very strong selective inhibitor in the immune response of the Th cell *in vitro* (see Baxevanis CN, et al., Immunogenetics (1980), 11, 617-625). Since the discovery of such monoclonal antibodies, they have been thought to be drugs that may be used for selective immunosuppression therapy for autoimmune diseases such as chronic rheumatism. Initial *in vivo* studies have revealed the useful effects of these monoclonal antibodies on Th cellular hetero- and autoimmune responses (see Rosenbaum JT. et al., J. Exp. Med. (1981), 154, 1694-1702; Waldor MK. et al., Proc. Natl. Acad. Sci. U.S.A. (1983), 80, 2713-2717; Jonker M. et al., J. Autoimmun. (1988), 1, 399-414; Stevens HP. et. al., Transplant. Proc. (1990), 22, 1783-1784). Further studies using primates have revealed that such monoclonal antibodies suppress graft versus host reactions in allogeneic transplants (Billing R. & Chatterjee S. (1983), Transplant. Proc., 15, 649-650; Jonker M. et. al., Transplant Proc. (1991), 23, 264-265). Currently, immunosuppressive agents such as ciclosporin A and FK506 are clinically used for suppressing rejection at the time of organ transplantation. These immunosuppressive agents are problematic in that they non-specifically suppress immune reactions so as to cause strong side effects. As disclosed in WO2003/033538, for example, a monoclonal antibody (anti-HLA-DR antibody) against HLA-DR, which is a type of major histocompatibility complex (MHC) class II (MHC) molecule, specifically suppresses HLA-DR-mediated immunoactivity. Thus, the anti-HLA-DR antibody is thought to be very useful. Accordingly, antibodies are appropriate for use as immunosuppressive agents that cause fewer side effects because of their features.

As described above, many antibodies suitable for therapeutic use have been studied, developed, and practically applied, so that the antibodies are more frequently used at medical sites. Under such current circumstances, a stable liquid medical formulation characterized by convenient operation for administration and preliminary work and a low risk of bacterial contamination, is required. In particular, a stable liquid medical formulation containing an antibody suitable for therapeutic use, such as an anti-HLA-DR antibody, is required in the art.
Patent Document 1: WO98/56418
Patent Document 2: JP Patent No. 2547556
Patent Document 3: WO97/04801
Non-patent document 1: Therapeutic Drug Carrier System vol. 10, No.4, 1993, pp. 307-377

### Disclosure of the Invention

### Problems to be solved by the invention

An object of the present invention is to provide a stable liquid medical formulation containing an antibody. Specifically, an object of the present invention is to provide a stable liquid medical formulation that contains a therapeutically effective amount of an antibody in a glutamate buffer sorbitol, polysorbate 80, and has a pH between 4.0 and 6.0.

### Means to solve the problems

As a result of intensive studies concerning such stable liquid medical formulation that contains a therapeutically effective amount of an antibody, the present inventors have succeeded in stabilization of the antibody contained in such liquid medical formulation. Thus, the present inventors have completed the present invention. The present invention provides:
[1]. A stable liquid medical formulation, which contains in a glutamate buffer a therapeutically effective amount of an antibody, a sorbitol as an isotonizing agent, and polysorbate 80 as a surfactant and has a pH between 4.0 and 6.0.
[2]. The stable liquid medical formulation according to [1], wherein the concentration of the buffer is between 1 mM and 50 mM.
[3]. The stable liquid medical formulation according to [1] or [2], which contains no salt as an isotonizing agent.
[4]. The stable liquid medical formulation according to any one of [1] to [3], wherein the osmotic pressure is between 250 mOsm and 350 mOsm.
[5]. The stable liquid medical formulation according to any one of [1] to [4], wherein the concentration of polysorbate 80 is between 0.02 mg/mL and 0.10 mg/mL.
[6]. The stable liquid medical formulation according to any one of [1] to [5], wherein the antibody is a human antibody, a humanized antibody, or a chimeric antibody.
[7]. The stable liquid medical formulation according to any one of [1] to [6], wherein the antibody is a monoclonal antibody.
[8]. The stable liquid medical formulation according to any one of [1] to [7], wherein the antibody is IgG.
[9]. The stable liquid medical formulation according to [8], wherein the IgG subclass is any one of IgG1, IgG2, or IgG4.
[10]. The stable liquid medical formulation according to [8] or [9], wherein the amino acid at position 331 (numbering based on the EU numbering system) in the heavy chain constant region has been substituted with Ser.
[11]. The stable liquid medical formulation according to any one of [1] to [10], wherein the antibody is an antibody against HLA-DR.
[12]. The stable liquid medical formulation according to any one of [1] to [11], wherein the antibody is an antibody against CD40.
[13]. The stable liquid medical formulation according to any one of [1] to [12], wherein the concentration of the antibody is between approximately 1 and 200 mg/mL.
[14]. A stable liquid medical formulation according to any one of [1] to [13], which has a pH between 4.5 and 6.0.
[15]. The stable liquid medical formulation according to any one of [1] to [14], which contains at least 1 type of stabilizing agent selected from the group consisting of glycine, methionine, cysteine hydrochloride, leucine, lysine hydrochloride, arginine hydrochloride, aspartic acid, ascorbic acid, EDTA, and salts thereof.

According to the present disclosure, a stable liquid medical formulation is provided, which contains a therapeutically effective amount of an antibody and glutamic acid for maintaining a pH between 4.0 and 6.0. The formulation is stable at least at a low temperature (2°C to 8°C) for 1 or more years. Specifically, the formulation is stable at least at 25°C for 3 months and/or at 40°C for 1 month. The formulation is also stable against freezing and thawing and vibration.

The present disclosure also relates to a product provided with a container that retains a stable liquid medical formulation containing a therapeutically effective amount of an antibody and a glutamate or citrate buffer for maintaining a pH between 4.0 and 6.0.

Furthermore, the present disclosure relates to a method for stabilizing an antibody in a liquid medical formulation, which comprises combining a therapeutically effective amount of an antibody and a glutamate buffer for maintaining a pH between 4.0 and 6.0.

In another aspect, the present disclosure relates to a therapeutic, preventive, or diagnostic method, which comprises administering a therapeutically effective amount of the liquid medical formulation disclosed herein to a mammal. When the antibody is an anti-HLA-DR antibody, examples of diseases to be prevented, treated, or diagnosed include tumors such as leukemia (including chronic lymphocytic leukemia and acute lymphocytic leukemia), lymphomas (including non Hodgkin's lymphoma, Hodgkin's lymphoma, T-cell lymphomas, B-cell lymphomas, Burkitt's lymphoma, malignant lymphoma, diffuse lymphoma, and follicular lymphoma), and myelomas (including multiple myeloma). Further examples of diseases to be prevented or treated include immunosuppression at the time of organ transplantation. (Rejection or GVHD at the time of transplantation of islets of the pancreas, kidney, liver, heart, or the like is to be prevented or treated.) Further examples of diseases to be prevented or treated include autoimmune diseases (e.g., rheumatism, arteriosclerosis, multiple sclerosis, systemic erythematodes, idiopathic thrombocythemia, and Crohn's disease), and allergies such as asthma.

These aspects are clearly understood by persons skilled in the art.

### Effect of the Invention

As described in examples, a formulation containing an antibody according to the invention of the present application is stable, even when the formulation is stored at 25°C or 40°C for 1 month. It is stable without increases in an aggregation, a degradation, a deamidation product, or an oxidation product of the antibody during such storage period. Furthermore, retention of the biological activity of the antibody was also confirmed. As shown in examples, the antibody in the formulation is maintained stably at a general storage temperature of 25°C or less by adjusting the pH of the liquid medical formulation between 4.0 and 6.0.

### Brief Description of the Drawings

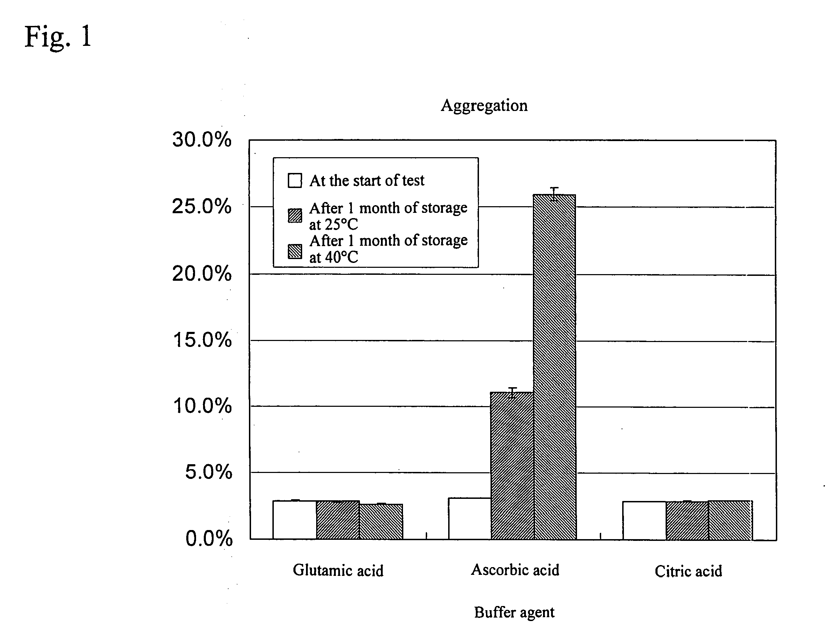

Fig. 1 shows changes in the amount of an aggregation when each liquid medical formulation containing an anti-HLA-DR antibody is stored at 25°C or 40°C. The amount of the aggregation was measured by size exclusion HPLC. The results show that a glutamate buffer formulation and a citrate buffer formulation were stable.

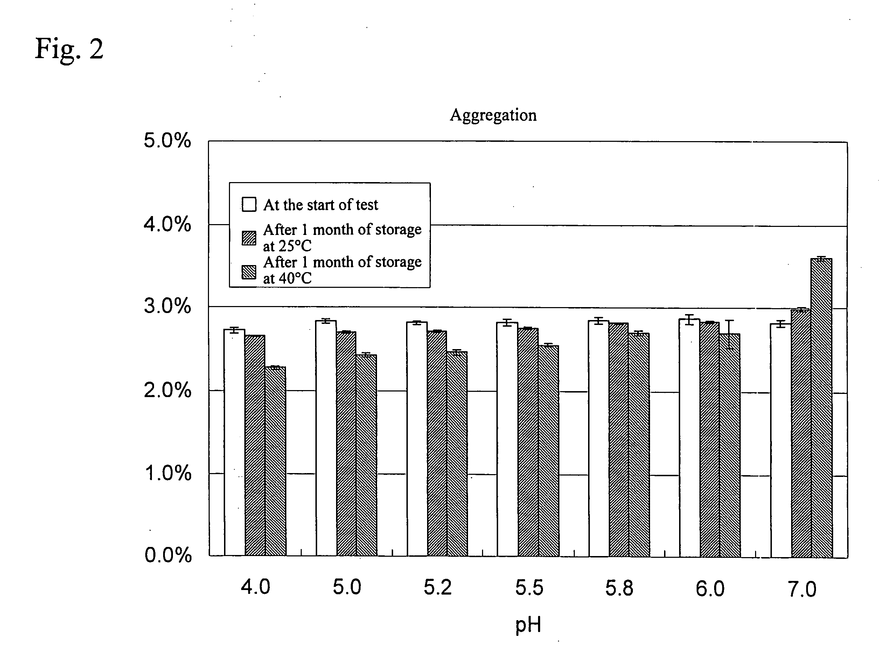

Fig. 2 shows changes in the amount of an aggregation when each liquid medical formulation containing an anti-HLA-DR antibody was stored at 25°C or 40°C. The amount of the aggregation was measured by size exclusion HPLC. The results show that a glutamate buffer formulation was stable at a pH between 4.0 and 6.0.

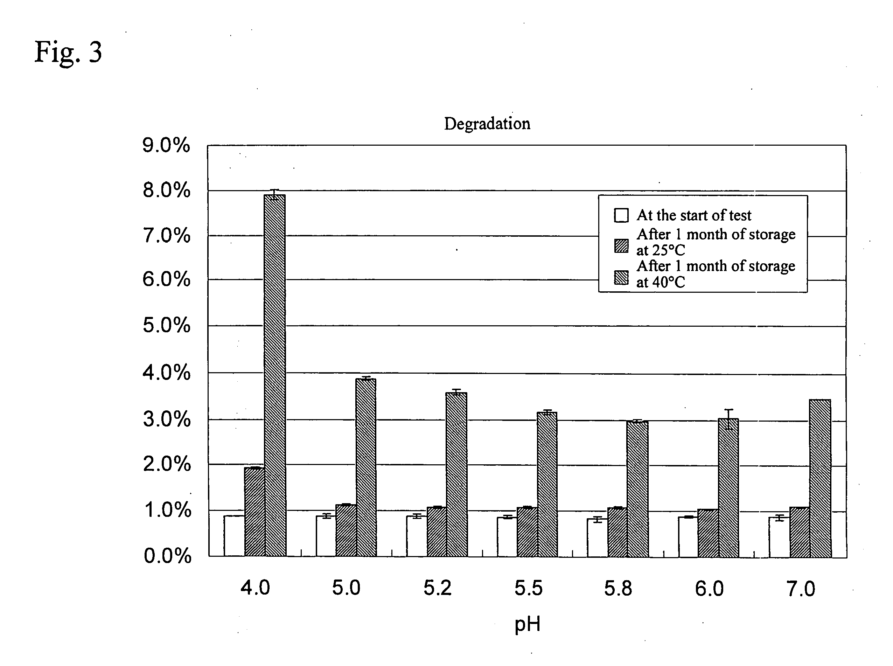

Fig. 3 shows changes in the amount of a degradation when each liquid medical formulation containing an anti-HLA-DR antibody was stored at 25°C or 40°C. The amount of the degradation was measured by size exclusion HPLC.

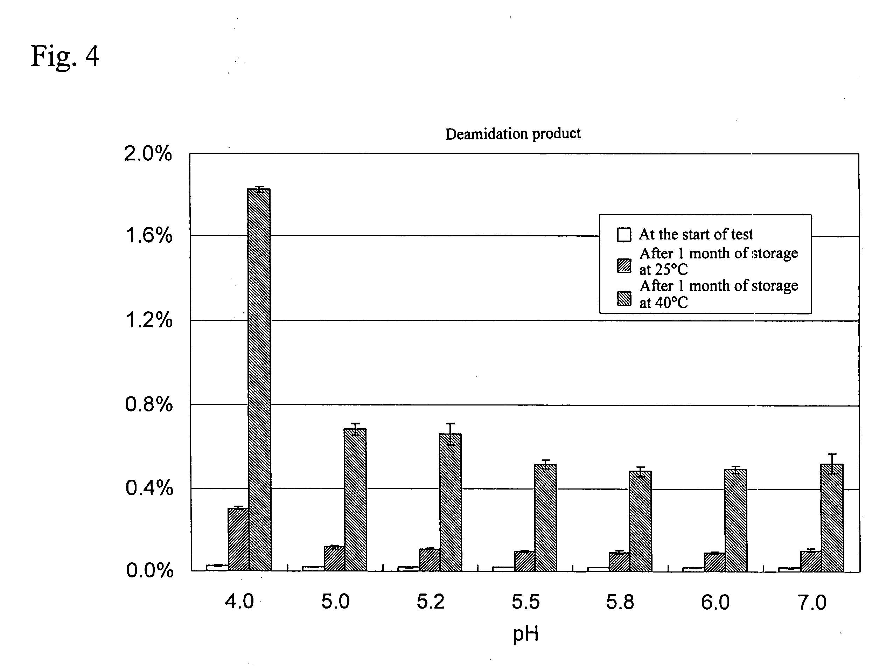

Fig. 4 shows changes in the amount of a deamidation product when each liquid medical formulation containing an anti-HLA-DR antibody was stored at 25°C or 40°C. The amount of the deamidation product was measured by cation exchange HPLC.

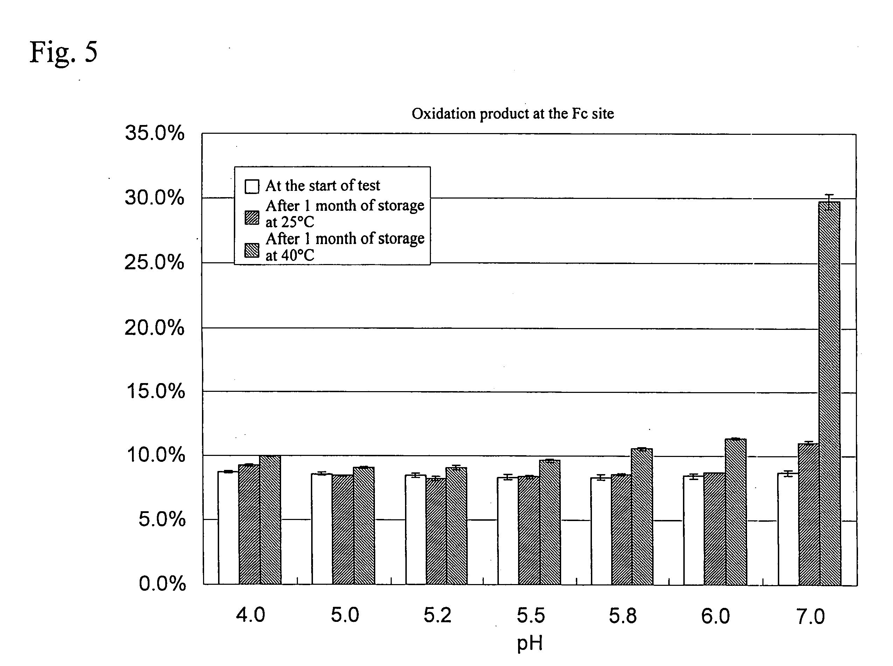

Fig. 5 shows changes in the amount of an oxidation product at an Fc site when each liquid medical formulation containing an anti-HLA-DR antibody was stored at 25°C or 40°C. The amount of the oxidation product at the Fc site was measured by hydrophobic HPLC. The results show that a glutamate buffer formulation was stable at a pH between 4.0 and 6.0.

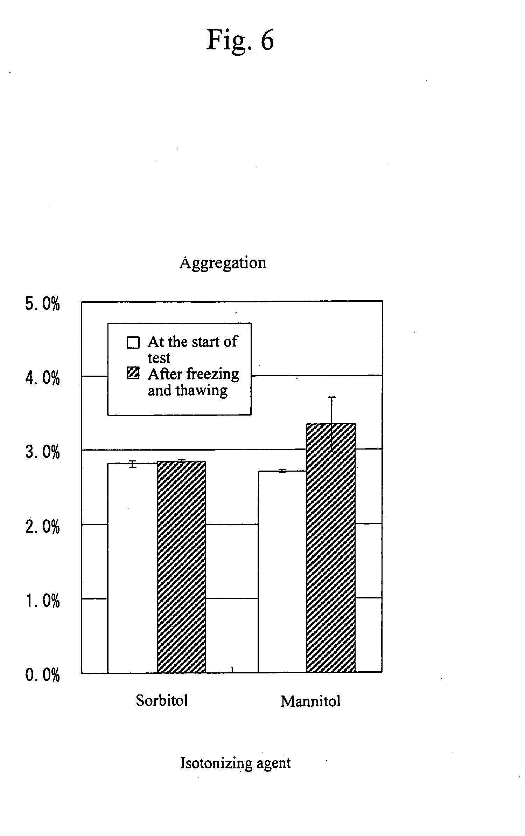

Fig. 6 shows changes in the amount of an aggregation after each liquid medical formulation containing an anti-HLA-DR antibody was repeatedly frozen and thawed. The amount of the aggregation was measured by size exclusion HPLC. The results show that a formulation containing a sorbitol as an isotonizing agent was stable.

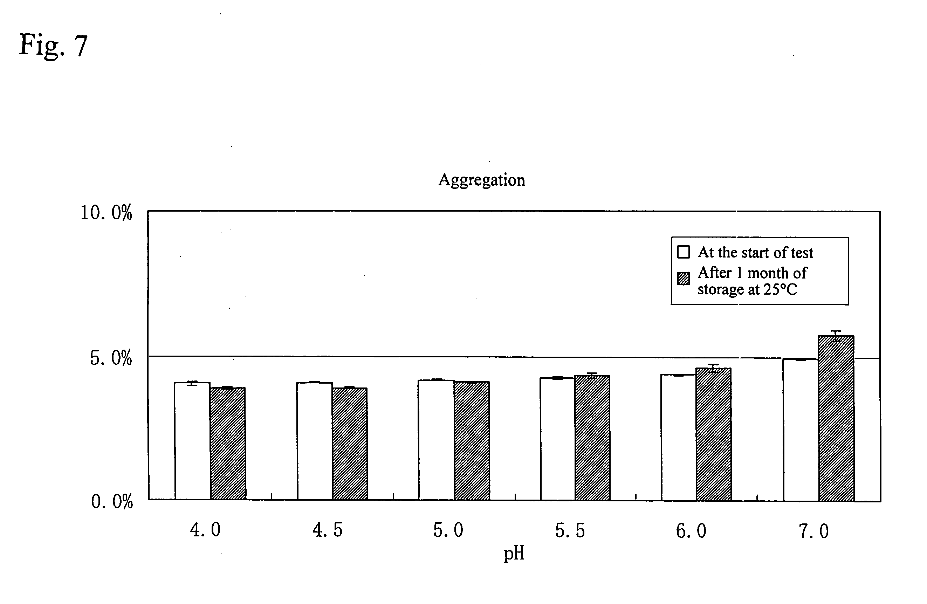

Fig. 7 shows changes in the amount of an aggregation when each liquid medical formulation containing an anti-CD40 antagonist antibody is stored at 25°C. The amount of the aggregation was measured by size exclusion HPLC. The results show that a glutamate buffer formulation was stable at a pH between 4.0 and 6.0.

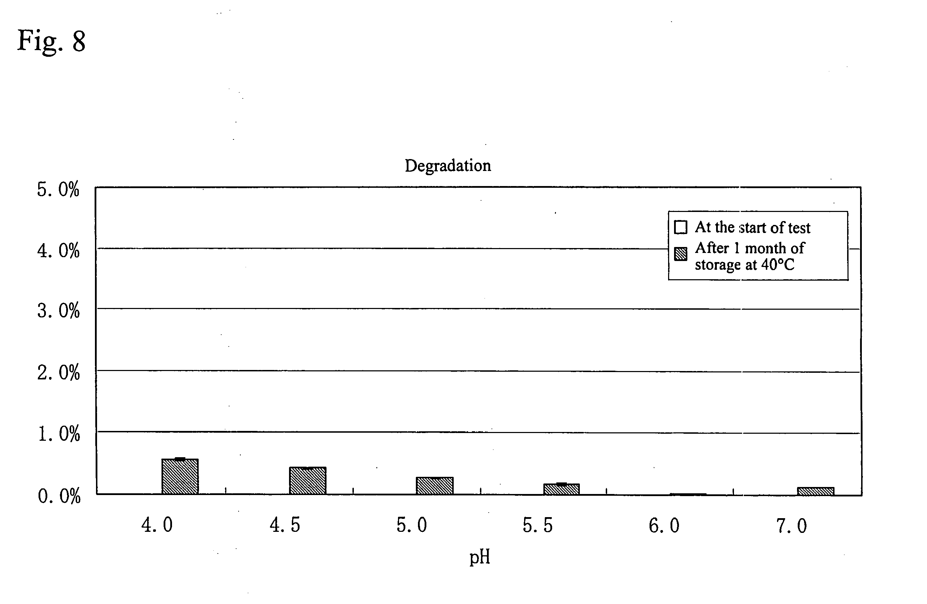

Fig. 8 shows changes in the amount of a degradation when each liquid medical formulation containing an anti-CD40 antagonist antibody is stored. The amount of the degradation was measured by size exclusion HPLC. The results show that a glutamate buffer formulation was stable at a pH between 4.0 and 7.0.

### Best Mode of Carrying out the Invention

The present invention is explained in detail as follows.

"Liquid medical formulation" included in this patent application means a formulation wherein: an active ingredient that is an antibody having medical effects is in the form of being dissolved in a liquid solution; the form enables the active ingredient to be clearly effective in the solution; and the formulation contains no additional ingredients that may disturb patient homeostasis to which the formulation is administered. Here, "enables the active ingredient to be clearly effective" means that the contained active ingredient maintains its activity and does not lose its medical effects.

"Additive other than the active ingredient, which does not disturb patient homeostasis as far as is possible" means a substance with safety that has been sufficiently confirmed by actual past therapeutic performance or a substance with safety that can be sufficiently predicted through evaluation of toxicity to cells or animals or by other methods even when the substance lacks actual past administration performance. Specifically, the liquid medical formulation of the present invention can contain an active ingredient that is an antibody having medical effects and other medically and pharmaceutically acceptable additives. Furthermore, "to maintain patient homeostasis" means that no additive other than the active ingredient has any biological activity that is unacceptable for a patient and/or that such additive is isotonic (essentially having the same osmotic pressure as that of human blood), if possible.

"Stable formulation" means a formulation wherein an active ingredient that retains its chemical and/or physical and/or biological stability during storage. Preferably, the formulation is stable at least at a low temperature (between 2°C and 8°C) for 1 or more years, specifically preferably at 25°C for at least 3 months and/or at 40°C for at least 1 month. Such formulation is also stable against freezing and thawing, light irradiation, and vibration. Protein stability is measured by various analysis methods. "Chemical stability" can be tested by detecting and quantifying the chemically altered state of a protein. Examples of chemical alterations include: size modification such as clipping that can be evaluated by size exclusion chromatography or SDS-PAGE; changes in electric charge (caused by deamidation, for example) that can be evaluated by ion exchange chromatography; and changes in hydrophilic/hydrophobic states (caused by oxidation, for example) that can be evaluated by hydrophobic chromatography. "Physical stability" means that there is no insoluble foreign matter and/or no turbidity and/or no aggregates are generated. Such physical stability can be evaluated by visual inspection of color and/or transparency and/or size exclusion chromatography. "Biological stability" can be evaluated by testing binding activity to an antigen. Such binding activity can be evaluated by size exclusion chromatography or ELISA, for example. When an antibody is not chemically or physically altered, the antibody retains its biological stability. Accordingly, when an antibody in a formulation retains its chemical stability and physical stability, it can be said that the formulation is stable. Specifically, whether or not a formulation is stable can be confirmed by measuring the presence or the absence of alterations of the contained antibody in terms of chemical and physical properties. In the case of a stable formulation, an aggregation, a degradation, a deamidation product, an oxidation product, or the like of the contained antibody does not increase during storage to a level that reduces the medical effects of the formulation. Furthermore, no insoluble foreign matter and no turbidity are observed. In the case of the stable formulation of the present invention, an aggregation, a degradation, a deamidation product, or an oxidation product of the contained antibody does not increase to a level that reduces the medical effects of the formulation, even when the formulation is stored at least at a low temperature (between 2°C and 8°C) for 1 or more years, at least at 25°C for 3 months, or at 40°C for 1 month. Furthermore, even when the formulation is frozen and thawed or vibrated, an aggregation, a degradation, a deamidation product, or an oxidation product of the contained antibody does not increase to a level that reduces the medical effects of the formulation. The aggregation of the antibody contained in the stable formulation of the present invention does not increase during storage. For example, when the formulation is stored at 25°C or 40°C for 1 month and such product is then measured by size exclusion HPLC, a small ratio of the aggregation to the antibody in the formulation is desired. Moreover, the degradation of the antibody contained in the stable formulation of the present invention does not greatly increase during storage. For example, when the formulation is stored at 25°C or 40°C for 1 month, a small ratio of the degradation to the antibody in the formulation is desired. Furthermore, the amount of the deamidation product of the antibody in the stable formulation of the present invention does not greatly increase during storage. For example, when the formulation is stored at 25°C or 40°C for 1 month, a small ratio of the deamidation product to the antibody in the formulation is desired. Moreover, the oxidation product at the Fc site of the antibody in the stable formulation of the present invention does not greatly increase during storage. For example, when the formulation is stored at 25°C or 40°C for 1 month, a small ratio of the oxidation product at the Fc site to the antibody in the formulation is desired.

"Therapeutically effective amount" of an antibody in the present invention indicates an amount effective for preventing or treating diseases, when the antibody is effective for treating such diseases. "Disease" means a disease with arbitrary symptoms regarding which the benefits can be obtained by treatment using an antibody. Examples of such disease include chronic and acute diseases or illness including pathological conditions that are predisposing factors for diseases of mammals. Such a therapeutically effective amount of an antibody existing in the formulation is determined in view of desirable dose and dosage form, for example. An illustrative antibody concentration in the formulation is between approximately 1 mg/mL and approximately 200 mg/mL, preferably between approximately 5 mg/mL and approximately 50 mg/mL, and most preferably approximately 10 mg/mL and/or approximately 20 mg/mL, for example.

An "antibody" included in this patent application is used in the broadest sense. In particular, examples of such antibody include a monoclonal antibody, a polyclonal antibody, a multi-specific antibody, and an antibody fragment, as long as the fragment retains desired biological activity.

The present invention relates to a stable liquid medical formulation containing an antibody. The antibody in the formulation is prepared using technology that can be used for antibody production in the art. Specifically, such technology involves the steps of (1) purifying a biopolymer to be used as an immunogen and/or preparing cells that excessively express an antigen protein to be used as an immunogen on the cell surfaces, (2) immunizing animals with such antigen by injecting it into the animals, collecting blood, assaying the antibody titer, determining the timing of excision of the spleen or the like, and then preparing antibody-producing cells, (3) preparing myeloma cells (myeloma), (4) fusing the antibody-producing cells to the myeloma, (5) selecting a hybridoma group producing a target antibody, (6) allowing division into single cell clones (cloning), (7) culturing the hybridoma for producing a monoclonal antibody in large amounts or raising animals into which the hybridoma has been transplanted, if necessary, (8) cloning a gene encoding a human monoclonal antibody from an antibody-producing cell such as the hybridoma, incorporating the resultant into an appropriate vector, introducing the vector into a host (e.g., mammalian cell lines, *Escherichia coli,* yeast cells, insect cells, or plant cells), and thus preparing a recombinant antibody produced with the use of gene recombination technology, (9) purifying the thus obtained antibody, and (10) examining physiological activity and the recognition specificity of the thus produced monoclonal antibody, testing the property of the antibody as a labeling reagent, or the like.

An example of such monoclonal antibody included herein comprises a heavy chain and/or a light chain having an amino acid sequence derived from each amino acid sequence of a heavy chain and/or a light chain composing an antibody by deletion, substitution, or addition of 1 or several amino acids. Such partial amino acid alteration (deletion, substitution, insertion, or addition) as described above can be conducted with reference to the amino acid sequence of an antibody contained in a formulation of the present invention by partially altering the nucleotide sequence encoding the amino acid sequence. Such partial alteration of a nucleotide sequence can be introduced by a standard method using known site-specific mutagenesis (Proc Natl Acad Sci U.S.A., 1984, Vol. 81: 5662). Here, "antibody" means an immunoglobulin wherein all regions including the heavy chain variable region, the heavy chain constant region, the light chain variable region, and the light chain constant region composing the immunoglobulin are derived from a gene encoding an immunoglobulin. Antibodies included in this patent application may be of any immunoglobulin classes and have isotypes. Examples of such antibodies further include altered antibodies with recombined subclasses and altered antibodies wherein amino acid at position 331 (numbered based on the EU numbering system (see Sequences of proteins of immunological interest, NIH Publication No. 91-3242)) in the heavy chain constant region has been substituted with Ser to give IgG1Ser or IgG2Ser. Furthermore, antibodies that are also included herein have further enhanced therapeutic effects against diseases such as cancer through binding with: a radionuclide such as iodine, yttrium, indium, or technetium (J. W. Goding, Momoclonal Antibodies: principles and practice, 1993 Academic Press); a bacterial toxin such as pyocyanic toxin, diphtheria toxin, or lysine; a chemotherapeutic such as methotrexate, mitomycin, or calicheamicin (D. J. King, Applications and Engineering of Monoclonal Antibodies., 1998 T. J. International Ltd.; M. L. Grossbard, Monoclonal Antibody-Based Therapy of Cancer, 1998 Marcel Dekker Inc.); or a prodrug such as Maytansinoid (Chari et al., Cancer Res., 1992, Vol. 52: 127; Liu et al., Proc. Natl. Acad. Sci. U.S.A., 1996, Vol. 93: 8681). Functional fragments of such antibodies are also included herein. "Functional fragment" means a part (partial fragment) of an antibody and means a fragment that retains 1 or more actions of an antibody on an antigen. Specific examples of such functional fragment include F(ab')₂, Fab', Fab, Fv, disulfide bond Fv, single chain Fv (scFv), diabody, and polymers thereof (D. J. King, Applications and Engineering of Monoclonal Antibodies, 1998, T. J. International Ltd.). Alternatively, "functional fragment" means a fragment of an antibody and can bind to an antigen. An antibody to be formulated is preferably essentially pure and is desirably essentially homogeneous (specifically, contains no contaminated protein or the like). An "essentially pure" antibody is one accounting for at least approximately 90% by weight and preferably at least approximately 95% by weight of the total weight of a composition that contains the antibody. An "essentially homogeneous" antibody is one accounting for at least approximately 99% by weight of the total weight of a composition that contains the antibody.

An antibody included in this patent application is preferably a human antibody, a humanized antibody, or a chimeric antibody. Furthermore, such antibody is preferably IgG, wherein preferably the IgG subclass is any one of IgG1, IgG2, or IgG4. Furthermore, IgG may have an amino acid sequence of a constant region, a part of which sequence has been subjected to amino acid deletion and/or substitution and/or insertion by partial gene alteration. Moreover, an antibody included herein is more preferably a human monoclonal antibody against HLA-DR. Further preferably, such an antibody is an anti-HLA-DR human monoclonal antibody disclosed in WO2003/033538, such as HD3, HD4, HD6, HD7, HD8, HD10, HD4G1, HD4G2Ser, HD4G4, HD8G1, HD8G1Ser, HD8G2, HD8G2Ser, or HD8G4. Of these antibodies, hybridomas producing HD4, HD6, HD8, and HD10 were internationally deposited under FERM BP-7771, FERM BP-7772, FERM BP-7773, and FERM BP-7774, respectively, on October 11, 2001, with the International Patent Organism Depositary (Tsukuba Central 6, 1-1-1 Higashi, Tsukuba, Ibaraki, Japan), the National Institute of Advanced Industrial Science and Technology). Furthermore, hybridomas producing HD3 and HD7 were internationally deposited under FERM BP-08534 and FERM BP-08536, respectively, on October 31, 2003, with the same International Patent Organism Depositary. Furthermore, this patent application further includes human monoclonal antibodies against CD40. Preferable examples of such monoclonal antibodies include: anti-CD40 antagonist antibodies disclosed in WO2002-088186, such as KM281-1-10, KM281-2-10-1-2, KM283-5, KM225-2-56, KM292-1-24, KM341-6-9, 4D11, 5H10, 11E1, 5G3, 3811, 3411, 3417, and F4-465; and anti-CD40 agonist antibodies such as KM302-1, KM341-1-19, KM643-4-11, 2053, 2105, 3821, 3822, 285, 110, 115, F1-102, F2-103, F5-77, and F5-157. Of these, hybridoma clones KM302-1, KM281-1-10, and KM281-2-10-1-2 were internationally deposited on May 9, 2001, under FERM BP-7578, FERM BP-7579, and FERM BP-7580, respectively; clones KM341-1-19 and 4D11 were internationally deposited on September 27, 2001, under FERM BP-7759 and FERM BP-7758, respectively; and clone 2105 was internationally deposited on April 17, 2002, under FERM BP-8024 with the International Patent Organism Depositary (Tsukuba Central 6, 1-1-1 Higashi, Tsukuba, Ibaraki, Japan), the National Institute of Advanced Industrial Science and Technology) under the Budapest Treaty. Furthermore, plasmids having heavy chain and light chain variable regions of F2-103, F5-77, and F5-157 were internationally deposited on April 19, 2001, under ATCC PTA-3302 (F2-103 heavy chain), ATCC PTA-3303 (F2-103 light chain), ATCC PTA-3304 (F5-77 heavy chain), ATCC PTA-3305 (F5-77 light chain), ATCC PTA-3306 (F5-157 heavy chain), and ATCC PTA-3307 (F5-157 light chain), respectively, and hybridoma clones F1-102 and F4-465 were internationally deposited on April 24, 2001, under ATCC PTA-3337 and ATCC PTA-3338, respectively, with the American Type Culture Collection, 10801 University Blvd., Manassas, Virginia, U.S.A. under the Budapest Treaty.

"Additives" included in the present invention include all ingredients contained in a liquid medical formulation other than the active ingredient. Examples of such additives include a buffer agent, a pH adjusting agent, an isotonizing agent, a stabilizing agent, a surfactant, an antiseptic agent, a suspension, and an emulsifier. Furthermore, an additive ingredient of a single type exhibiting 2 or more types of effects is also included.

"Buffer agent" indicates an additive that functions to moderate changes in pH with the use of the action of acid-base conjugated ingredients. A buffer of the present invention preferably has a pH range between approximately 4.0 and approximately 6.0, more preferably between approximately 4.5 and approximately 6.0, further preferably between approximately 4.0 and approximately 5.0, and even further preferably between approximately 4.5 and approximately 5.0. Alternatively, such buffer has a pH range between approximately 5.0 and approximately 6.0, preferably between 5.2 and 5.8, and further preferably has a pH of approximately 5.5. When a buffer having a pH within such pH range is used, any antibody can be stable at 25°C or lower, which is a general storage temperature for medical formulations. In addition, depending on antibody types, such stable pH range may be varied at a higher temperature, such as 40°C. For example, a buffer solution that is used when an anti-HLA-DR antibody is used preferably has a pH between approximately 5.0 and 6.0, more preferably between 5.2 and 5.8, and further preferably a pH of approximately 5.5. A buffer solution that is used when an anti-CD40 antagonist antibody is used preferably has a pH range between approximately 4.0 and 6.0, more preferably between approximately 4.5 and 6.0, and further preferably between approximately 4.0 and approximately 5.0. Examples of a buffer agent having a pH that is adjusted within such pH range include glutamate (e.g., sodium glutamate), acetate (e.g., sodium acetate), succinate (e.g., sodium succinate), gluconate, citric acid, citrate, ascorbate (e.g., sodium ascorbate), and other organic acid buffers. When a stable formulation is desired in terms of freezing and thawing, preferably, a buffer agent is not phosphate. In the present disclosure, a buffer agent is preferably glutamate. The concentration of a buffer agent is between approximately 1 mM and approximately 50 mM, preferably between 5 mM and 20 mM, and is most suitably approximately 10 mM, depending on buffering ability and/or desired osmotic pressure.

"Isotonizing agent" indicates an agent with which a subject formulation is prepared to have osmotic pressure that is essentially the same as that of human blood. An isotonic formulation has osmotic pressure between approximately 250 and 350 mOsm and the ratio of its osmotic pressure to that (determined to be 1) of physiological saline is approximately 1:1. As isotonizing agents, generally, salts or/and a polyol are used. An isotonizing agent of the present disclosure preferably essentially contains no salt but preferably contains polyol.

"Polyol" means a substance having a plurality of hydroxyl groups. Examples of polyol include a sugar (reducing or nonreducing sugar), a sugar alcohol, and saccharic acid. A suitable polyol that is used herein has a molecular weight of approximately less than 600 kD (e.g., within a range between approximately 120 kD and approximately 400 kD). "Reducing sugar" means a sugar capable of reducing metal ions or a sugar containing a hemiacetal group capable of reacting covalently with lysine within a protein and other amino groups. "Nonreducing sugar" has no such properties. Examples of such reducing sugar include fructose, mannose, maltose, lactose, arabinose, xylose, ribose, rhamnose, galactose, and glucose. Examples of such nonreducing sugar include sucrose, trehalose, sorbose, melezitose, and raffinose. Examples of sugar alcohol include mannitol, xylitol, erythritol, threitol, sorbitol, and glycerol. Examples of saccharic acid include L-gluconic acid and its metal salt. A preferable polyol is a nonreducing sugar that does not have any chemical effects on an antibody in a solution. When it is desired that a formulation be stable in terms of freezing and thawing, a suitable polyol is not crystallized at a freezing temperature (e.g., -20°C) that destabilizes an antibody within a formulation. A sorbitol is preferable because of its excellent solution stability.

Examples of a "surfactant" include nonionic surfactants such as polysorbate (e.g., polysorbate 20 and 80) and poloxamer (e.g., poloxamer 188). The amount of a surfactant to be added herein is an amount that reduces the aggregation of formulated antibodies and/or minimizes particle formation in a formulation and/or reduces adsorption. Examples of a surfactant in the present disclosure preferably include polysorbate and more preferably polysorbate 80. A surfactant may be present in a formulation preferably at a concentration between 0.02 mg/mL and 0.10 mg/mL and most preferably at a concentration of approximately 0.05 mg/mL.

"Stabilizing agent" means an additive that further enhances the chemical and/or physical and/or biological stability of an active ingredient during storage through being added in a small amount, preferably at 10 mM or less. The formulation of the present invention may contain a stabilizing agent. Such stabilizing agent is selected from the group consisting of glycine, methionine, cysteine hydrochloride, leucine, lysine hydrochloride, arginine hydrochloride, aspartic acid, ascorbic acid, EDTA, and salts thereof, for example.

A formulation used for *in vivo* administration must be sterile. This can easily be achieved by filtration (by which formulations are caused to pass through sterile filtration membranes) before or after preparation of the formulation.

A formulation is administered to a mammal or preferably a human who needs treatment using an antibody by a known method in a bolus dose or by continuous injection over a certain period of time, for example. Specifically, a formulation is administered via an intravenous, intramuscular, intraperitoneal, intracerebral, subcutaneous, intraarticular, intrasynovial, intrasubarachnoidal, oral, local, or inhalation route. In a preferred embodiment, a formulation is intravenously administered to a mammal. For such purpose, a formulation is injected using a syringe, for example, or via an intravenous drip infusion line.

In another embodiment of the present disclosure, a product provided with a container that contains the liquid formulation of the present invention is provided. If necessary, instructions for the use of such container are also provided. Examples of a container that is suitable herein include a bottle, a vial, an ampule, and a syringe. Such container can be formed using various materials such as glass or plastic. An illustrative container is a 3-mL to 20-mL glass vial. Such container for storing a formulation has a label on the container or includes a label with the container. Such label shows instructions for use. Such product may also contain another buffer agent, a diluent, a filter, a needle, a syringe, and a packaged insert with instructions for use. Furthermore, such product may further contain other materials desirable in terms of marketing and in view of users.

### Examples

The present invention is further sufficiently understood by referring to the following examples. However, these examples are not intended to limit the scope of the invention.

### Example 1 Liquid formulations containing anti-HLA-DR antibody (examination of buffer agent)

This example describes liquid formulations each containing an antibody (anti-HLA-DR antibody) against HLA-DR disclosed in WO2003/033538.

In this example, formulations listed in Table 1 were prepared and then the effects of buffer agent types on antibody stability were evaluated.

**Table 1 List of formulations subjected to this study**

| | Concentration of active ingredient | Buffer agent | Isotonizing agent | Surfactant | pH |
|---|---|---|---|---|---|
| 1 | 10 mg/mL anti-HLA-DR antibody | 10 mM glutamic acid | 262 mM D-sorbitol | 0.05 mg/mL polysorbate 80 | 5.5 |
| 2 | | 10 mM ascorbic acid | | | |
| 3 | | 10 mM citric acid | | | |

### (1) Preparation of formulation samples

Reagents used in this examination were the anti-HLA-DR antibody (approximately 18 mg/mL, prepared according to the method disclosed in WO2003/0033538 at the CMC R&D Laboratories, Kirin Brewery Co., Ltd.), sodium L-glutamate, monohydrate (The Japanese Pharmaceutical Codex), L-histidine (Japanese Pharmacopoeia), sodium citrate, monohydrate (Japanese Pharmacopoeia), ascorbic acid (Japanese Pharmacopoeia), D-sorbitol (Japanese Pharmacopoeia), D-mannitol (Japanese Pharmacopoeia), hydrochloric acid (Japanese Pharmacopoeia), polysorbate 80 (Japanese Pharmacopoeia), and water for injection (Japanese Pharmacopoeia).

A placebo solution containing no drug substance was previously prepared for each formulation sample. Each formulation sample was prepared by substituting the formulation placebo solution with an anti-HLA-DR antibody solution using an NAP column (produced by Pharmacia Biotech). Protein concentrations were adjusted through conversion using the coefficient of ε = 1.4 at absorption of OD280 nm.

Each formulation sample was subjected to sterile filtration through a 0.22-µm filter (produced by Millipore Corporation) within a clean bench. 5-mL glass vials (conforming to Japanese Pharmacopoeia) were each packed with 1 mL of the sample within a clean bench while keeping sterility. Furthermore, solutions (placebo) containing no anti-HLA-DR antibody (for dilution of samples to be analyzed and for blank samples for analysis) were subjected to sterile filtration through a 0.22-µm bottle top filter (NALGENE, produced by Nalge Nunc International Corporation) within a clean bench.

### (2) Test conditions

Each formulation sample was subjected to stress according to the following conditions so as to perform stability evaluation in this example.
Thermostability test: Formulation samples were stored in an incubator (produced by TABAI ESPEC) controlled at 40°C or 25°C for 1 month.

Furthermore, each sample was stored in a low-temperature container controlled at 4°C until the start of analysis after each instance of provision of stress.

### (3) Analysis method

Size exclusion HPLC test (SEC): Aggregation contents and degradation contents were calculated by the size exclusion high-speed liquid chromatography method. If necessary, a sample was diluted at 1 mg/mL followed by injection of 15 µL of the diluted sample at an atmospheric temperature. Separation was performed using a TSKgel G3000 SWXL 30 cm × 7.8 mm (produced by TOSOH CORPORATION) column and 20 mM sodium phosphate and 500 mM sodium chloride at pH 7.0 as mobile phase at a flow rate of 0.5 mL/minute for 30 minutes of analysis time. Detection was performed at 215 nm. In addition, products eluted before the major peak were defined as aggregations and products eluted after the major peak were defined as degradations. Cation exchange HPLC test (IEC): Deamidation product contents were calculated by the cation exchange high-performance liquid chromatography method. 60 µL of a sample that had been appropriately diluted at 5 mg/mL was injected. TSKgel BIOAssist S (produced by TOSOH CORPORATION) was used as a separation column and detection was performed at 280 nm. 20 mM tartaric acid at pH 4.5 was used as mobile phase A and 20 mM tartaric acid and 1 M sodium chloride at pH 4.5 were used as mobile phase B. Analysis was conducted under the most suitable gradient conditions. In addition, deteriorated products eluted before the major peak were defined as deamidation products. Hydrophobic HPLC test (HIC): Oxidation product (at the Fc site) contents were calculated by the hydrophobic chromatography method. To 250 µL of a sample that had been appropriately diluted at 1 mg/mL, 250 mM sodium phosphate, 12.5 mM L-cysteine, 20 µL of a liquid solution at pH 7.0, and 2.5 µL of a 2.5 mg/mL papain solution were added, followed by 2 hours of enzyme treatment at 37°C. Thus, an Fc fragment sample was prepared. 15 µL of the thus prepared Fc fragment sample was injected. TSKgel Butyl-NPR 3.5 cm × 4.6 mm (produced by TOSOH CORPORATION) was used as a separation column. Analysis and detection were performed at 215 nm. 20 mM Tris (hydroxymethyl) aminomethane at pH 7.0 was used as mobile phase A and 20 mM Tris (hydroxymethyl) aminomethane and 2M ammonium sulfate at pH 7.0 were used as mobile phase B. Analysis was conducted under the most suitable gradient conditions. In addition, deteriorated products eluted before the major peak were defined as oxidation products.
SDS-PAGE test: If necessary, a test solution was diluted at 200 µg/mL. A Tris SDS sample treatment solution (produced by Daiichi Pure Chemicals Co., Ltd.) was added to the sample solution in a volume half of that of the sample solution, thereby preparing a nonreduction sample solution. Furthermore, the thus obtained sample solution was diluted if necessary. A Tris SDSβME sample treatment solution (produced by Daiichi Pure Chemicals Co., Ltd.) was added to the sample solution in a volume half of that of the sample solution, followed by 15 minutes of heating at 65°C. Thus a reduction sample solution was prepared. An electrophoresis bath was filled with a Tris/glycine/SDS buffer (produced by Bio-Rad Laboratories) for electrophoresis. 5 µL of each sample solution was applied to 4%-20% polyacrylamide gel (produced by Daiichi Pure Chemicals Co., Ltd.). Electrophoresis was performed with a constant current of approximately 20 mA until the blue of bromophenol blue contained in each sample solution migrated into the vicinity of the lower end portion of the gel. After electrophoresis, gel was silver-stained and then detection was performed. In addition, to determine approximate molecular weights of samples and deteriorated products, molecular weight markers (including 200 kDa, 116.2 kDa, 66.3 kDa, 42.4 kDa, 30.0 kDa, and 17.2 kDa proteins) were electrophoresed simultaneously.
Insoluble foreign matter and turbidity: The presence or the absence of insoluble foreign matter and turbidity was visually determined at a position directly below a white light source with level of light of approximately 5000 lux.
Osmotic pressure ratio: Osmotic pressure was measured using an automated osmotic pressure meter (OSMO STATION OM-6050 produced by ARKRAY, Inc.). The ratio of the osmotic pressure to the simultaneously measured osmotic pressure of physiological saline was calculated.
pH: pH measurement was performed using an automated pH meter (e.g., MP-230 produced by Mettler-Toledo International Inc.). At the start of measurement, calibration was performed using standard solutions with pH 4, pH 7, and pH 9 and then pH measurement was performed.

### (4) Results

Fig. 1 shows changes in the aggregation amount when each liquid medical formulation was stored at 25°C or 40°C. The amount of the aggregation was measured by size exclusion HPLC. The results show that the glutamate buffer formulation and the citrate buffer formulation were stable. Furthermore, in terms of other factors for analysis (degradation, deamidation product, and oxidation product at the Fc site), the ascorbate buffer was unstable, although the glutamate buffer formulation and the citrate buffer formulation were stable.

According to visual inspection, the formulation containing an ascorbate buffer was colored yellow or brown after storage. The glutamate and citrate buffer formulations were stable such that no changes were observed before or after storage. Insoluble fine particles were observed to a slight extent in all samples, and the samples were stable such that no changes were observed before or after storage. Furthermore, regarding pH measured upon storage at 25°C or 40°C, a tendency of shifting to the lower pH side was observed in the ascorbate buffer formulation. The glutamate and citrate buffer formulations were stable, such that no changes were observed before or after storage. In all the samples, the osmotic pressure ratio of each sample to physiological saline was always 1:1. No changes were observed before or after provision of stress.

It was revealed that the formulation containing an ascorbate buffer used therein was very unstable because of its significantly high levels of many indices indicating deterioration as revealed by measurement.

It was demonstrated by this example that glutamic acid or citric acid is suitable as a buffer agent for stabilizing an antibody. Most preferably, glutamic acid is used as a buffer agent.

### Example 2 Liquid formulations containing anti-HLA-DR antibody (examination of pH)

This example describes liquid formulations each containing an antibody against HLA-DR (anti-HLA-DR antibody) disclosed in WO2003/033538.

In this example, formulations listed in Table 2 were prepared and then suitable formulation pH was evaluated in detail.

**Table 2 List of formulations subjected to this study**

| | Concentration of active ingredient | Buffer agent | Isotonizing agent | Surfactant | pH |
|---|---|---|---|---|---|
| 1 | 10 mg/mL anti-HLA-DR antibody | 10 mM glutamic acid | 262 mM D-sorbitol | 0.05 mg/mL polysorbate 80 | 4.0 |
| 2 | | | | | 5.0 |
| 3 | | | | | 5.2 |
| 4 | | | | | 5.5 |
| 5 | | | | | 5.8 |
| 6 | | | | | 6.0 |
| 7 | | | | | 7.0 |

### (1) Materials and methods

Materials and analysis methods employed in this example are the same as those described in Example 1.

### (2) Test conditions

Each formulation sample was subjected to stress according to the following conditions so as to perform stability evaluation in this example.
Thermostability test: Formulation samples were stored in an incubator (produced by TABAI ESPEC) controlled at 40°C or 25°C for 1 month.
Freezing and thawing test: Each formulation sample was stored alternately in a freezer at -20°C and a low-temperature container at 4°C, so that freezing and thawing were repeated 3 times. Thus, samples were prepared. In addition, whether each sample was completely frozen or thawed was confirmed by visual inspection at every cycle.
Forced vibration test: The test was conducted for 20 minutes using a vibration tester (RECIPRO SHAKER SR-II produced by Taiyo Scientific Industrial Co., Ltd.) under vibration conditions of 300 rpm and 40 mm, so that samples were prepared. Photostability test: Under a white fluorescent lamp controlled to have approximately 4000 lux, each sample was irradiated with light so as to achieve a level of light of approximately 1,200,000 lux.

### (3) Results

Fig. 2 shows changes in the amount of an aggregation when each liquid medical formulation was stored at 25°C or 40°C. The amount of the aggregation was measured by size exclusion HPLC. The results show that the glutamate buffer formulation was stable at a pH between 4.0 and 6.0. Furthermore, Fig. 3 shows changes in the amount of a degradation when each liquid medical formulation was stored at 25°C or 40°C. The amount of the degradation was measured by size exclusion HPLC. The results show that the glutamate buffer formulation was stable at a pH between 4.0 and 7.0 when the formulation was stored at 25°C. Furthermore, in SDS-PAGE analysis, electrophoresis images were obtained, with which a tendency similar to that exhibited in the results obtained by size exclusion HPLC could be confirmed. Fig. 4 shows changes in the amount of a deamidation product when each liquid medical formulation was stored at 25°C or 40°C. The amount of the deamidation product was measured by cation exchange HPLC. The results show that the glutamate buffer formulation was stable at a pH between 4.0 and 7.0 when the formulation was stored at 25°C. Fig. 5 shows changes in the amount of an oxidation product at an Fc site when each liquid medical formulation was stored at 25°C or 40°C. The amount of the oxidation product at the Fc site was measured by hydrophobic HPLC. The results show that the glutamate buffer formulation was stable at a pH between 4.0 and 6.0. Specifically, the glutamate buffer formulation was stable such that almost no foreign matter, turbidity, or insoluble foreign matter were observed by visual inspection and almost no changes in osmotic pressure ratios or pHs were observed before or after provision of stress.

As a result of the photostability test, in all the samples, increases in the amounts of the aggregation, the degradation, the deamidation product, and the oxidation product at the Fc site were observed after light irradiation. These changes can be suppressed with light shielding using a paper box. Thus, the formulations can be stably stored by taking some light-shielding measures during storage. In addition, the formulations were stable such that no changes were observed as a result of freezing and thawing or before or after provision of vibration stress in terms of all factors for analysis.

### Example 3 Formulations containing anti-HLA-DR antibody (examination of isotonizing agent)

This example describes liquid formulations each containing an antibody (anti-HLA-DR antibody) against HLA-DR disclosed in WO2003/033538.

In this example, formulations listed in Table 3 were prepared and then the effects of isotonizing agents in the formulations on the stability of the formulations were evaluated.

**Table 3 List of formulations subjected to this study**

| | Concentration of active ingredient | Buffer agent | Isotonizing agent | Surfactant | pH |
|---|---|---|---|---|---|
| 1 | 10 mg/mL anti-HLA-DR antibody | 10 mM glutamic acid | 262 mM sorbitol | 0.05 mg/mL polysorbate 80 | 5.5 |
| 2 | | | 262 mM mannitol | | |

### (1) Materials and methods

Materials and analysis methods employed in this example are the same as those described in Example 1.

### (2) Test conditions

Samples were subjected to stress in a manner that was the same as that in the method described in Example 2.

### (3) Results and consideration

Fig. 6 shows changes in the amount of an aggregation after each liquid medical formulation was repeatedly frozen and thawed. The amount of the aggregation was measured by size exclusion HPLC. The results show that the formulation containing sorbitol as an isotonizing agent was stable. A sorbitol is not crystallized even at the time of freezing. However, mannitol is crystallized at the time of freezing, so that it may particularly physically disrupt antibody molecules at the time of freezing. It was demonstrated that as a polyol that is used as an isotonizing agent, a substance such as a sorbitol that is not crystallized at the time of freezing is more suitable. Regarding other test conditions, no differences were observed in terms of stability. Furthermore, the inventors conducted a preliminary examination; that is, a test for comparing a salt (sodium chloride) as an isotonizing agent with a sugar (sorbitol) as an isotonizing agent. In a thermostability test, the present inventors obtained knowledge that formulations are more stable in terms of the amounts of aggregations, degradations, and deamidation products generated, when sugars are used.

Therefore, the isotonizing agent of the present disclosure preferably contains essentially no salt. A preferable polyol is a nonreducing sugar that does not have any chemical effects on an antibody in a solution. When it is desired that a formulation be stable in terms of freezing and thawing, a suitable isotonizing agent is not crystallized at a freezing temperature (e.g., -20°C) that destabilizes an antibody in the formulation. A sorbitol is preferable because it has excellent solution stability.

Furthermore, the present inventors have also used polysorbate 80 as a surfactant and studied the effects of its concentration on antibody stability. As a result, addition of the surfactant reduced the aggregation of the formulated antibody. However, a tendency was observed, whereby the amount of the oxidation product at the Fc site of the antibody was increased by the addition of an excessive amount (0.20 mg/mL or more) of polysorbate 80. Hence, it is preferable that a surfactant be present in a formulation at a concentration between preferably 0.02 mg/mL and 0.10 mg/mL and most preferably approximately 0.05 mg/mL

Moreover, the present inventors conducted a preliminary examination; that is, an examination concerning the addition of a stabilizing agent (glycine, methionine, cysteine hydrochloride, leucine, lysine hydrochloride, arginine hydrochloride, aspartic acid, ascorbic acid, or EDTA), in addition to the above examples. For example, a further effect of suppressing insoluble fine particles was observed with the addition of glycine. Furthermore, an effect of methionine in suppressing the generation of an oxidation product was obtained. Accordingly, the present invention may also contain these stabilizing agents.

Furthermore, the present inventors have also conducted a similar examination using a recombinant complete human IgG1 antibody (anti-HSA antibody) against HSA. Thus, the present inventors have confirmed that the present invention can be successfully performed with any antibody types. Moreover, the present inventors have examined liquid medical formulations using the anti-HSA antibody. Specifically, the present inventors have examined the formulations with antibody concentrations ranging from 1 mg/mL to 100 mg/mL. Thus, it has been revealed that the methods revealed by the present invention can be successfully performed with any antibody concentrations.

### Example 4 Preparation of medical formulations containing antibody

Examples of preparing stable liquid medical formulations containing antibodies are as described below.

CHO cells were transformed with a gene encoding an antibody (HD3, HD4, HD6, HD7, HD8, HD10, HD4G1, HD4G2Ser, HD4G4, HD8G1, HD8G1Ser, HD8G2, HD8G2Ser, or HD8G4) used in the present invention so as to be able to express and produce the antibody. Each of these antibodies was purified by the method disclosed in WO2003/033538 from the culture supernatants of the CHO cells. A thus purified antibody was concentrated at approximately 10 mg/mL, followed by substitution with a 10-mM glutamate buffer, 262-mM D-sorbitol, and a buffer with a pH of 5.5 using an ultrafiltration membrane. The antibody contained in a solution collected after buffer substitution was concentrated and prepared at an antibody concentration of 20 mg/mL. Polysorbate 80 was added at 0.05 mg/mL, so that a liquid medical formulation containing the antibody at 20 mg/mL was obtained. Moreover, the above 20 mg/mL antibody formulation was diluted with a liquid medical formulation (10 mM glutamate buffer, 262-mM D-sorbitol, and 0.05-mg/mL polysorbate 80 solution) containing no antibodies. Thus, liquid medical formulations with any antibody concentrations were prepared. Table 4 (A to N) below lists examples of the compositions of each liquid medical formulation (1 mL) containing antibodies at a concentration of 10 mg/mL.

**Table 4**

| A Composition of HD3-containing liquid medical formulation | |
|---|---|
| HD3 | 10 mg |
| Sodium glutamate, monohydrate | 1.87 mg |
| Hydrochloric acid | 0.057 mg |
| D-sorbitol | 47.73 mg |
| Polysorbate 80 | 0.05 mg |
| Distilled water for injection | (1 mL) |
| | |

| B Composition of HD4-containing liquid medical formulation | |
|---|---|
| HD4 | 10 mg |
| Sodium glutamate, monohydrate | 1.87 mg |
| Hydrochloric acid | 0.057 mg |
| D-sorbitol | 47.73 mg |
| Polysorbate 80 | 0.05 mg |
| Distilled water for injection | (1 mL) |
| | |

| C Composition of HD6-containing liquid medical formulation | |
|---|---|
| HD6 | 10 mg |
| Sodium glutamate, monohydrate | 1.87 mg |
| Hydrochloric acid | 0.057 mg |
| D-sorbitol | 47.73 mg |
| Polysorbate 80 | 0.05 mg |
| Distilled water for injection | (1 mL) |
| | |

| D Composition of HD7-containing liquid medical formulation | |
|---|---|
| HD7 | 10 mg |
| Sodium glutamate, monohydrate | 1.87 mg |
| Hydrochloric acid | 0.057 mg |
| D-sorbitol | 47.73 mg |
| Polysorbate 80 | 0.05 mg |
| Distilled water for injection | (1 mL) |
| | |

| E Composition of HD8-containing liquid medical formulation | |
|---|---|
| HD8 | 10 mg |
| Sodium glutamate, monohydrate | 1.87 mg |
| Hydrochloric acid | 0.057 mg |
| D-sorbitol | 47.73 mg |
| Polysorbate 80 | 0.05 mg |
| Distilled water for injection | (1 mL) |
| | |

| F Composition of HD10-containing liquid medical formulation | |
|---|---|
| HD10 | 10 mg |
| Sodium glutamate, monohydrate | 1.87 mg |
| Hydrochloric acid | 0.057 mg |
| D-sorbitol | 47.73 mg |
| Polysorbate 80 | 0.05 mg |
| Distilled water for injection | (1 mL) |
| | |

| G Composition of HD4G1-containing liquid medical formulation | |
|---|---|
| HD4G1 | 10 mg |
| Sodium glutamate, monohydrate | 1.87 mg |
| Hydrochloric acid | 0.057 mg |
| D-sorbitol | 47.73 mg |
| Polysorbate 80 | 0.05 mg |
| Distilled water for injection | (1 mL) |
| | |

| H Composition of HD4G2ser-containing liquid medical formulation | |
|---|---|
| HD4G2ser | 10 mg |
| Sodium glutamate, monohydrate | 1.87 mg |
| Hydrochloric acid | 0.057 mg |
| D-sorbitol | 47.73 mg |
| Polysorbate 80 | 0.05 mg |
| Distilled water for injection | (1 mL) |
| | |

| I Composition of HD4G4-containing liquid medical formulation | |
|---|---|
| HD4G4 | 10 mg |
| Sodium glutamate, monohydrate | 1.87 mg |
| Hydrochloric acid | 0.057 mg |
| D-sorbitol | 47.73 mg |
| Polysorbate 80 | 0.05 mg |
| Distilled water for injection | (1 mL) |
| | |

| J Composition of HD8G1-containing liquid medical formulation | |
|---|---|
| HD8G1 | 10 mg |
| Sodium glutamate, monohydrate | 1.87 mg |
| Hydrochloric acid | 0.057 mg |
| D-sorbitol | 47.73 mg |
| Polysorbate 80 | 0.05 mg |
| Distilled water for injection | (1 mL) |
| | |

| K Composition of HD8G1 ser-containing liquid medical formulation | |
|---|---|
| HD8G1 ser | 10 mg |
| Sodium glutamate, monohydrate | 1.87 mg |
| Hydrochloric acid | 0.057 mg |
| D-sorbitol | 47.73 mg |
| Polysorbate 80 | 0.05 mg |
| Distilled water for injection | (1 mL) |
| | |

| L Composition of HD8G2-containing liquid medical formulation | |
|---|---|
| HD8G2 | 10 mg |
| Sodium glutamate, monohydrate | 1.87 mg |
| Hydrochloric acid | 0.057 mg |
| D-sorbitol | 47.73 mg |
| Polysorbate 80 | 0.05 mg |
| Distilled water for injection | (1 mL) |
| | |

| M Composition of HD8G2ser-containing liquid medical formulation | |
|---|---|
| HD8G2ser | 10 mg |
| Sodium glutamate, monohydrate | 1.87 mg |
| Hydrochloric acid | 0.057 mg |
| D-sorbitol | 47.73 mg |
| Polysorbate 80 | 0.05 mg |
| Distilled water for injection | (1 mL) |
| | |

| N Composition of HD8G4-containing liquid medical formulation | |
|---|---|
| HD8G4 | 10 mg |
| Sodium glutamate, monohydrate | 1.87 mg |
| Hydrochloric acid | 0.057 mg |
| D-sorbitol | 47.73 mg |
| Polysorbate 80 | 0.05 mg |
| Distilled water for injection | (1 mL) |

The thus obtained liquid medical formulations were sterilized using sterile filtration membranes. With the use of an automated filling machine under sterile conditions or the like, previously sterilized vials were filled with these formulations. Each vial was sealed with a rubber stopper and then an aluminum cap was screwed on tightly, so that a sterile antibody-containing liquid medical formulation was obtained.

### Example 5 Liquid formulations containing anti-CD40 antibody

This example describes liquid formulations each containing an antibody (anti-CD40 antagonist antibody) against CD40 disclosed in WO 02/088186.

In this example, formulations listed in Table 5 were prepared and then the effect of pH on the stability of the antibody was evaluated.

**Table 5 List of formulations subjected to this study**

| | Active ingredient | Buffer agent | Isotonizing agent | Surfactant | pH |
|---|---|---|---|---|---|
| 1 | 10 mg/mL anti-CD40 antagonist antibody | 10 mM glutamic acid | 262 mM D-sorbitol | 0.05 mg/mL polysorbate 80 | 4.0 |
| 2 | | | | | 4.5 |
| 3 | | | | | 5.0 |
| 4 | | | | | 5.5 |
| 5 | | | | | 6.0 |
| 6 | | | | | 7.0 |

### Preparation of formulation samples

Reagents used in this examination were the anti-CD40 antagonist antibody (approximately 15 mg/mL, prepared according to the method disclosed in WO 02-088186 at the CMC R&D Laboratories, Kirin Brewery Co., Ltd.), sodium L-glutamate, monohydrate (The Japanese Pharmaceutical Codex), D-sorbitol (Japanese Pharmacopoeia), sodium hydroxide (Japanese Pharmacopoeia), hydrochloric acid (Japanese Pharmacopoeia), polysorbate 80 (Japanese Pharmacopoeia), and water for injection (Japanese Pharmacopoeia).

A placebo solution containing no drug substance was previously prepared for each formulation sample. Each formulation sample was prepared by substituting the formulation placebo solution with an anti-CD40 antagonist antibody using an NAP column (produced by Pharmacia Biotech). Protein concentrations were adjusted through conversion using the coefficient ε = 1.65 at absorption of OD280 nm.

Each formulation sample was subjected to sterile filtration through a 0.22-µm filter (produced by Millipore Corporation) within a clean bench. 5-mL glass vials (FUJI GLASS CO., LTD., White U-KB2CS (no coating)) were each packed with 1 mL of the sample within a clean bench while keeping sterility. Furthermore, solutions (placebo) containing no anti-CD40 antagonist antibody (for dilution of samples to be analyzed and for blank samples for analysis) were subjected to sterile filtration through a 0.22-µm bottle top filter (NALGENE, produced by Nalge Nunc International Corporation) within a clean bench.

### Test conditions

Each formulation sample was subjected to stress according to the following conditions so as to perform stability evaluation in this example.
Thermostability test: Formulation samples were stored in an incubator (produced by TABAI ESPEC) controlled at 40°C or 25°C for 1 month.

Furthermore, each sample was stored in a low-temperature container controlled at 4°C until the start of analysis after each instance of provision of stress.

### Analysis method

Size exclusion HPLC test (SEC): Aggregation contents and degradation contents were calculated by the size exclusion high-speed liquid chromatography method. If necessary, a sample was diluted at 1 mg/mL followed by injection of 15 µL of the diluted sample at an atmospheric temperature. Separation was performed using a TSKgel G3000 SWXL 30 cm X 7.8 mm (produced by TOSOH CORPORATION) column and 20 mM sodium phosphate and 500 mM sodium chloride at pH 7.0 as mobile phase at a flow rate of 0.5 mL/minute for 30 minutes of analysis time. Detection was performed at 215 nm. In addition, products eluted before the major peak were defined as aggregations and products eluted after the major peak were defined as degradations.
SDS-PAGE test: If necessary, a test solution was diluted at 200 µg/mL. A Tris SDS sample treatment solution (produced by Daiichi Pure Chemicals Co., Ltd.) was added to the sample solution in a volume half of that of the sample solution, thereby preparing a nonreduction sample solution. Furthermore, the thus obtained sample solution was diluted if necessary. A Tris SDSβME sample treatment solution (produced by Daiichi Pure Chemicals Co., Ltd.) was added to the sample solution in a volume half of that of the sample solution, followed by 15 minutes of heating at 65°C. Thus a reduction sample solution was prepared. An electrophoresis bath was filled with a Tris/glycine/SDS buffer (produced by Bio-Rad Laboratories) for electrophoresis. 5 µL of each sample solution was applied to 4%-20% polyacrylamide gel (produced by Daiichi Pure Chemicals Co., Ltd.). Electrophoresis was performed with a constant current of approximately 20 mA until the blue of bromophenol blue contained in each sample solution migrated into the vicinity of the lower end portion of the gel. After electrophoresis, gel was silver-stained and then detection was performed. In addition, to determine approximate molecular weights of samples and deteriorated products, molecular weight markers (including 200.0 kDa, 116.2 kDa, 66.3 kDa, 42.4 kDa, 30.0 kDa, and 17.2 kDa proteins) were electrophoresed simultaneously.
Osmotic pressure ratio: Osmotic pressure was measured using an automated osmotic pressure meter (OSMO STATION OM-6050 produced by ARKRAY, Inc.). The ratio of the osmotic pressure to the simultaneously measured osmotic pressure of physiological saline was calculated.
pH: pH measurement was performed using an automated pH meter (e.g., MP-230 produced by Mettler-Toledo International Inc.). At the start of measurement, calibration was performed using standard solutions with pH 4, pH 7, and pH 9 and then pH measurement was performed.

### Results and consideration

Fig. 7 shows the aggregation amount when each liquid medical formulation was stored at 25°C. The amount of the aggregation was measured by size exclusion HPLC. The results show that the liquid medical formulations were stable at a pH between 4.0 and 6.0. Fig. 8 shows the amount of a degradation when each liquid medical formulation was stored at 40°C. The amount of the degradation was measured by sizes exclusion HPLC. The degradations were detected in trace amounts regardless of pHs. Furthermore, in all the samples, pHs measured upon storage at 25°C or 40°C were stable such that no changes in pH were observed before or after storage. In all the samples, the osmotic pressure ratio to that of physiological saline was always approximately 1:1. No changes were observed before or after provision of stress.

## Claims

1. A stable liquid medical formulation, which contains in a glutamate buffer a therapeutically effective amount of an antibody, a sorbitol as an isotonizing agent, and polysorbate 80 as a surfactant and has a pH between 4.0 and 6.0.

2. The stable liquid medical formulation according to claim 1, wherein the concentration of the buffer is between 1 mM and 50 mM.

3. The stable liquid medical formulation according to claim 1 or 2, which contains no salt as an isotonizing agent.

4. The stable liquid medical formulation according to any one of claims 1 to 3, wherein the osmotic pressure is between 250 mOsm and 350 mOsm.

5. The stable liquid medical formulation according to any one of claims 1 to 4, wherein the concentration of polysorbate 80 is between 0.02 mg/mL and 0.10 mg/mL.

6. The stable liquid medical formulation according to any one of claims 1 to 5, wherein the antibody is a human antibody, a humanized antibody, or a chimeric antibody.

7. The stable liquid medical formulation according to any one of claims 1 to 6, wherein the antibody is a monoclonal antibody.

8. The stable liquid medical formulation according to any one of claims 1 to 7, wherein the antibody is IgG.

9. The stable liquid medical formulation according to claim 8, wherein the IgG subclass is any one of IgG1, IgG2, or IgG4.

10. The stable liquid medical formulation according to claim 8 or 9, wherein the amino acid at position 331 (numbering based on the EU numbering system) in the heavy chain constant region has been substituted with Ser .

11. The stable liquid medical formulation according to any one of claims 1 to 10, wherein the antibody is an antibody against HLA-DR.

12. The stable liquid medical formulation according to any one of claims 1 to 11, wherein the antibody is an antibody against CD40.

13. The stable liquid medical formulation according to any one of claims 1 to 12, wherein the concentration of the antibody is between approximately 1 and 200 mg/mL.

14. A stable liquid medical formulation according to any one of claims 1 to 13, which has a pH between 4.5 and 6.0.

15. The stable liquid medical formulation according to any one of claims 1 to 14, which contains at least 1 type of stabilizing agent selected from the group consisting of glycine, methionine, cysteine hydrochloride, leucine, lysine hydrochloride, arginine hydrochloride, aspartic acid, ascorbic acid, EDTA, and salts thereof.

## Patentansprüche

1. Stabile flüssige medizinische Zubereitung, die in einem Glutamatpuffer eine therapeutisch wirksame Menge eines Antikörpers, ein Sorbitol als ein isotonisierendes Mittel und Polysorbat 80 als ein Tensid enthält und einen pH zwischen 4,0 und 6,0 aufweist.

2. Stabile flüssige medizinische Zubereitung gemäß Anspruch 1, worin die Konzentration des Puffers zwischen 1 mM und 50 mM beträgt.

3. Stabile flüssige medizinische Zubereitung gemäß Anspruch 1 oder 2, die kein Salz als isotonisierendes Mittel enthält.

4. Stabile flüssige medizinische Zubereitung gemäß einem der Ansprüche 1 bis 3, worin der osmotische Druck zwischen 250 mOsm und 350 mOsm beträgt.

5. Stabile flüssige medizinische Zubereitung gemäß einem der Ansprüche 1 bis 4, worin die Konzentration des Polysorbats 80 zwischen 0,02 mg/ml und 0,10 mg/ml beträgt.

6. Stabile flüssige medizinische Zubereitung gemäß einem der Ansprüche 1 bis 5, worin der Antikörper ein humaner Antikörper, ein humanisierter Antikörper oder ein chimärer Antikörper ist.

7. Stabile flüssige medizinische Zubereitung gemäß einem der Ansprüche 1 bis 6, worin der Antikörper ein monoklonaler Antikörper ist.

8. Stabile flüssige medizinische Zubereitung gemäß einem der Ansprüche 1 bis 7, worin der Antikörper IgG ist.

9. Stabile flüssige medizinische Zubereitung gemäß Anspruch 8, worin die IgG-Unterklasse eine beliebige aus IgG1, IgG2 oder IgG4 ist.

10. Stabile flüssige medizinische Zubereitung gemäß Anspruch 8 oder 9, worin die Aminosäure an der Position 331 (wobei die Nummerierung auf dem EU-Nummerierungssystem basiert) in der konstanten Region der schweren Kette mit Ser substituiert worden ist.

11. Stabile flüssige medizinische Zubereitung gemäß einem der Ansprüche 1 bis 10, worin der Antikörper ein Antikörper gegen HLA-DR ist.

12. Stabile flüssige medizinische Zubereitung gemäß einem der Ansprüche 1 bis 11, worin der Antikörper ein Antikörper gegen CD40 ist.

13. Stabile flüssige medizinische Zubereitung gemäß einem der Ansprüche 1 bis 12, worin die Konzentration des Antikörpers zwischen ungefähr 1 und 200 mg/ml beträgt.

14. Stabile flüssige medizinische Zubereitung gemäß einem der Ansprüche 1 bis 13, die einen pH zwischen 4,5 und 6,0 aufweist.

15. Stabile flüssige medizinische Zubereitung gemäß einem der Ansprüche 1 bis 14, die zumindest einen Typ eines Stabilisierungsmittels enthält, das aus der Gruppe ausgewählt ist, bestehend aus Glycin, Methionin, Cysteinhydrochlorid, Leucin, Lysinhydrochlorid, Argininhydrochlorid, Asparaginsäure, Ascorbinsäure, EDTA und Salzen davon.

## Revendications

1. Formulation médicale liquide stable, qui contient dans un tampon de glutamate une quantité thérapeutiquement efficace d'un anticorps, un sorbitol comme agent d'isotonicité et du polysorbate 80 comme agent tensioactif, et a un pH compris entre 4,0 et 6,0.

2. Formulation médicale liquide stable selon la revendication 1, dans laquelle la concentration du tampon est comprise entre 1 mM et 50 mM.

3. Formulation médicale liquide stable selon la revendication 1 ou 2, qui ne contient pas de sel comme agent d'isotonicité.

4. Formulation médicale liquide stable selon l'une quelconque des revendications 1 à 3, dans laquelle la pression osmotique est comprise entre 250 mOsm et 350 mOsm.

5. Formulation médicale liquide stable selon l'une quelconque des revendications 1 à 4, dans laquelle la concentration du polysorbate 80 est comprise entre 0,02 mg/ml et 0,10 mg/ml.

6. Formulation médicale liquide stable selon l'une quelconque des revendications 1 à 5, dans laquelle l'anticorps est un anticorps humain, un anticorps humanisé ou un anticorps chimère.

7. Formulation médicale liquide stable selon l'une quelconque des revendications 1 à 6, dans laquelle l'anticorps est un anticorps monoclonal.

8. Formulation médicale liquide stable selon l'une quelconque des revendications 1 à 7, dans laquelle l'anticorps est une IgG.

9. Formulation médicale liquide stable selon la revendication 8, dans laquelle la sous-classe d'IgG est l'un quelconque parmi l'IgG1, l'IgG2 ou l'IgG4.

10. Formulation médicale liquide stable selon la revendication 8 ou 9, dans laquelle l'acide aminé en position 331 (numérotation selon le système de numérotation de l'UE) dans la région constante de la chaine lourde a été substitué par une Ser.

11. Formulation médicale liquide stable selon l'une quelconque des revendications 1 à 10, dans laquelle l'anticorps est un anticorps contre HLA-DR.

12. Formulation médicale liquide stable selon l'une quelconque des revendications 1 à 11, dans laquelle l'anticorps est un anticorps dirigé contre CD40.

13. Formulation médicale liquide stable selon l'une quelconque des revendications 1 à 12, dans laquelle la concentration de l'anticorps est comprise entre environ 1 et 200 mg/ml.

14. Formulation médicale liquide stable, selon l'une quelconque des revendications 1 à 13, qui a un pH compris entre 4,0 et 6,0.

15. Formulation médicale liquide stable, selon l'une quelconque des revendications 1 à 14, qui contient au moins 1 type d'agent stabilisant choisi dans le groupe constitué par la glycine, la méthionine, le chlorhydrate de cystéine, la leucine, le chlorhydrate de lysine, le chlorhydrate d'arginine, l'acide aspartique, l'acide ascorbique, l'EDTA et les sels de ceux-ci.
